# EUROPEAN PATENT APPLICATION

(11) **EP 2 281 806 A1**
(43) Date of publication of application: **09.02.2011**
(21) Application number: 09176527.1
(22) Date of filing: 19.11.2009
(51) Int. Cl.: C07C 231/24, C07C 237/46, B01J 13/04

(54) **Process for isolating iodixanol from an aqueous solution**

(30) Priority: 21.07.2009 US 227105 P
(71) Applicant: GE Healthcare AS, 0401 Oslo (NO)
(72) Inventor: Askildsen, Arne, 4521 Spangereid (NO)
(74) Representative: Wulff, Marianne Weiby

(57) **Abstract**

This invention relates to the isolation of iodixanol (1,3-bis(acetamido)-N,N'-bis[3,5-bis(2,3- dihydroxypropylaminocarbonyl)-2,4,6-triiodophenyl]-2-hydroxypropane), more specifically to the isolation of iodixanol from an aqueous solution, by spray granulation.

## Description

### TECHNICAL FIELD

This invention relates to the isolation of iodixanol (1,3-bis(acetamido)-N,N'-bis[3,5-bis(2,3-dihydroxypropylaminocarbonyl)-2,4,6-triiodophenyl]-2-hydroxypropane), more specifically to the isolation of iodixanol from an aqueous solution.

### BACKGROUND OF THE INVENTION

Iodixanol is the non-proprietory name of the chemical drug substance of a non-ionic X-ray contrast agent marketed under the trade name Visipaque™. Visipaque™ is one of the most used agents in diagnostic X-ray procedures and is manufactured in large quantities.

The manufacture of such non-ionic X-ray contrast agents involves the production of the chemical drug substance (referred to as primary production) followed by formulation into the drug product (referred to as secondary production). Primary production of iodixanol involves a multi step chemical synthesis and a thorough purification process. For a commercial drug product it is important for the primary production to be efficient and economical and to provide a drug substance fulfilling the specifications, e.g. as expressed in the US Pharmacopea.

A number of methods are known for the preparation of iodixanol. These are all multi step chemical synthetic processes and the cost of the final formulated product thus mainly depends on these processes. It is therefore important to optimize the processes both for economic and environmental reasons.

In the last purification step in the production process for iodixanol the product from the final crystallization is dissolved in water to be able to remove traces of residual impurities, e.g. ionic compounds, colored substances and residual solvents. The final step in the process is evaporation and drying of the purified product from an aqueous syrup. This is a challenging process since it involves a phase transition from solution to solid state.

A conventional technique for the challenging final step is tumble drying under reduced pressure and at elevated temperature. This process is time consuming and usually requires 1-2 days in order to obtain a dry powder.

Another technique is conventional spray drying. However this process results in a powder of very low density that requires large warehouse areas for storage of the substance before formulation.

WO 2007/116039 describes a process for the preparation of the non-ionic X-ray contrast agent ioxilan by atomization, for example by spray drying.

It is hence a desire to identify alternative drying processes that are feasible in an industrial scale and that solve the problems with the conventional prior art methods.

We have now surprisingly found that if spray drying is combined with granulation a product of acceptable powder density can be obtained at short cycle times and hence fulfill one or more of the desired improvements listed above.

### SUMMARY OF THE INVENTION

The present invention provides a large scale process for isolating 1,3-bis(acetamido)-N,N'-bis[3,5-bis(2,3-dihydroxypropylaminocarbonyl)-2,4,6-triiodophenyl]-2-hydroxypropane from an aqueous solution.

Thus the invention provides a process for isolating 1,3-bis(acetamido)-N,N'-bis[3,5-bis(2,3-dihydroxypropylaminocarbonyl)-2,4,6-triiodophenyl]-2-hydroxypropane from an aqueous solution by using spray granulation.

The instant process is simple, fast and provides a product with sufficient purity and suitable physical properties to make the manufacturing process of iodixanol economically feasible in an industrial scale.

### DETAILED DESCRIPTION OF THE INVENTION

The final step in the conventional process of manufacturing iodixanol is evaporation and drying of the purified product.

We have now surprisingly found that isolation of iodixanol can be achieved by using spray granulation resulting in a product that meets the requirements in order to make the overall process of manufacturing iodixanol feasible.

Thus the invention provides a process for isolating 1,3-bis(acetamido)-N,N'-bis[3,5-bis(2,3-dihydroxypropylaminocarbonyl)-2,4,6-triiodophenyl]-2-hydroxypropane from an aqueous solution by using spray granulation.

The process of the present invention provides isolated iodixanol with high powder density that minimizes the required storage area before formulation. Also, very short cycle times result in high production capacity.

Additional advantages with the process of the invention are also identified. The thermal stress on the product is substantially lower compared to tumble drying since the time at high temperature is very short in a spray granulator compared to in a tumble dryer. The residence time in a spray granulator is seconds or minutes compared to days in a tumble dryer. This leads to a substantially smaller risk of formation of byproducts during the drying process, e.g. liberation of free inorganic iodide. Also, the risk of formation of traces of crystalline iodixanol is low due to the short time at elevated temperature in the spray granulator compared to the tumble dryer. Formation of a crystalline product is a kinetic process that is more pronounced at high temperatures, and leads to longer dissolution times and hence longer cycle times in the subsequent formulation process.

Any spray granulator can be used in the process according to the present invention.

Preferably the spray granulation is performed at a temperature from about 55 to 80°C, and in any event at a temperature less than about 100°C in order not to risk decomposing the product. However, the air inlet temperature is usually higher than the temperature the product is exposed to, and may be up to about 130°C. The feed flow rate and the inlet temperature are appropriately adjusted in order not to exceed the decomposition temperature at the outlet. The concentration of the solution fed to the spray granulator is typically about 0.8 to 1.3 kg iodixanol per liter solution, and most preferably about 1.0-1.2 kg iodixanol per liter solution.

The bulk density of the granulate resulting from the process of the present invention is typically about 0.8 to 1.0 kg/l, and the particle sizes typically range from about 20 to 1500 µm with the majority of the particles having sizes of about 200 to 300 µm.

The content of crystalline material in the dry product is typically less than 0.1 w/w % and the content of free inorganic iodide is typically not more than about 2 µg/g.

The invention is illustrated further by the following example that is not to be construed as limiting the invention in scope to the specific procedures or products described in them.

### EXAMPLE 1

5400 liter of an aqueous solution containing about 2770 kg iodixanol at 30°C is fed to a spray granulator. The granulation output is about 400 kg/hour, giving a total drying time of about 7 hours. The bulk density of the granulate is about 0.8-1.0 kg/L with particle sizes of 20-1500 µm, with a maximum at about 200-300 µm. The product temperature in the granulation process lies between 55 and 80°C. The water content in the granulate is about 2 w/w %. The content of crystalline material in the dry solid is less than 0.1 w/w % and the content of free inorganic iodide typically not more than 2 µg/g.

All patents, journal articles, publications and other documents discussed and/or cited above are hereby incorporated by reference.

## Claims

1. Process for isolating 1,3-bis(acetamido)-N,N'-bis[3,5-bis(2,3-dihydroxypropylaminocarbonyl)-2,4,6-triiodophenyl]-2-hydroxypropane from an aqueous solution by spray granulation.
